# EUROPÄISCHE PATENTSCHRIFT

(11) **EP 1 032 560 B1**
(45) Veröffentlichungstag und Bekanntmachung des Hinweises auf die Patenterteilung: **29.12.2004**
(21) Anmeldenummer: 98963476.1
(22) Anmeldetag: 17.11.1998
(51) Int. Cl.: A61K 31/4015, A61K 31/403, A61P 7/00

(54) **AN 4-STELLUNG SUBSTITUIERTE 2-PYRROLIDINON-DERIVATE ZUR VERRINGERUNG DES EXTRAZELLULÄREN GLUTAMAT-SPIEGELS**
POSITION-4 SUBSTITUTED 2-PYRROLIDINONE DERIVATIVES TO REDUCE THE LEVEL OF EXTRACELLULAR GLUTAMATE
DERIVES DE 2-PYRROLIDINONE SUBSTITUES EN POSITION 4, UTILISES POUR REDUIRE LE NIVEAU DE GLUTAMATE EXTRACELLULAIRE

(30) Priorität: 18.11.1997 DE 19751062
(43) Veröffentlichungstag der Anmeldung: 06.09.2000
(60) Teilanmeldung: 04022320.8
(73) Patentinhaber: KLINIKUM DER ALBERT-LUDWIGS-UNIVERSITÄT FREIBURG, D-79106 Freiburg (DE)
(72) Erfinder: FEUERSTEIN, Thomas, J., D-79289 Horben (DE); KNÖRLE, Rainer, D-79117 Freiburg (DE)
(74) Vertreter: Best, Michael, Dr.
(86) Internationale Anmeldenummer: PCT/EP1998/007383
(87) Internationale Veröffentlichungsnummer: WO 1999/025683

(56) Entgegenhaltungen:
- EP-A- 0 414 263
- EP-A- 0 446 570
- WO-A-97/29101
- WO-A-97/33858
- WO-A-97/33859
- A. ENDERS ET AL.: ARZNEIMITTEL-FORSCHUNG - DRUG RESEARCH, Bd. 10, Nr. 4, 1960, Seiten 243-50, XP002099344 in der Anmeldung erwähnt
- J. NAKAMURA ET AL.: J. PHARMACOBIO-DYN., Bd. 14, 1991, Seiten 1-8, XP002099345 in der Anmeldung erwähnt
- P. A. REDDY ET AL.: JOURNAL OF MEDICINAL CHEMISTRY, Bd. 39, Nr. 9, 1996, Seiten 1898-1906, XP002099346 in der Anmeldung erwähnt
- M. P REZ DE LA MORA ET AL.: BIOCHEMICAL PHARMACOLOGY, Bd. 22, 1973, Seiten 2635-9, XP002099347 in der Anmeldung erwähnt

## Beschreibung

Glutamat (Glu) ist der wesentliche exzitatorische Transmitter im Zentralnervensystem. Hohe extrazelluläre Konzentrationen an Glu im Extrazellulärraum führen zu exzitotoxischen Schädigungen (Siesjö BK, Bengtsson F, Grampp W, Theander S, 1989, Calcium, excitotoxins, and neuronal death in the brain. Ann N Y Acad Sci 568:234-251). Beispiele solcher Erkrankungen des Zentralnervensystems, bei denen die Exzitotoxizität eine Rolle spielt, sind Schlaganfall, Hypoglykämie, Hypoxie, Trauma und Epilepsie als akute Störungen, aber auch chronische Störungen im Sinne der Neurodegeneration wie die Alzheimer'sche Erkrankung, die Demenz bei AIDS, die Amyotrophe Lateralsklerose, der Morbus Parkinson, der chronische Alkoholismus und andere. Bei chronischen Schmerzen ist eine erhöhte glutamaterge Transmission (einhergehend mit erhöhter extrazellulärer Glu-Konzentration) für plastische Veränderungen verantwortlich und wesentlich an der Pathogenese der von der eigentlichen Ursache entfernten "Schmerzkrankheit" beteiligt (Liu HT, Mantyh PW, Bäsbaum AI, 1997, NMDA-receptor regulation of substance P release from primary afferent nociceptors. Nature 386:721-724).

Substanzen, welche die Exzitotoxizität von und die plastische Veränderungen durch Glu durch Verringerung der extrazellulären Glu-Spiegel verhindern, wären für die Therapie und Prophylaxe der genannten Krankheitsbilder von entscheidendem Vorteil.

Mehrere Substanzen, die (angeblich) die glutamaterge Transmission beeinflussen, sind bekannt oder als Medikamente bereits auf dem Markt. Zu ihnen gehören der Glu-Freisetzungshemmer Riluzol (Bryson HM, Fulton B, Benfield P, 1996, Riluzole. A review of its pharmacodynamic and pharmacokinetic properties and therapeutic potential in amyotrophic lateral sclerosis. Drugs 52:549-563) und Lamotrigin. Letzteres wirkt jedoch trotz ursprünglich gegenteiliger Behauptungen nicht als spezifischer Hemmer der Glu-Ausschüttung (Waldmeier PC, Martin P, Stocklin K, Portet C, Schmutz M, 1996, Effect of carbamazepine, oxcarbazepine and lamotrigine on the increase in extracellular glutamate elicited by veratridine in rat cortex and striatum. Naunyn Schmiedeberg's Arch Pharmacol 354:164-172). Auch das GABA-Derivat Gabapentin soll im millimolaren Konzentrationsbereich die Glu-Synthese hemmen (Goldlust A, Su TZ, Welty DF, Taylor CP, Oxender DL, 1995, Effects of anticonvulsant drug gabapentin on the enzymes in metabolic pathways of glutamate and GABA. Epilepsy Res 22:1-11); diese Konzentrationen sind jedoch *in vivo* nicht erreichbar.

Einige 1-, 3- und 5-substituierte Derivate des 2-Pyrrolidinons sind als antikonvulsiv wirksame und/oder möglicherweise die glutamaterge Transmission beeinflussende Substanzen bekannt (Nakamura J, Miwa T, Mori Y, Sasaki H, Shibasaki J, 1991, Comparative studies on the anticonvulsant activity of lipophilic derivatives of gamma-aminobutyric acid and 2-pyrrolidinone in mice. J Pharmacobiodyn 14:1-.8; Reddy PA, Hsiang BC, Latifi TN, Hill MW, Woodward KE, Rothman SM, Ferrendelli JA, Covey DF, 1996, 3,3-Dialkyl- and 3-alkyl-3-benzyl-substituted 2-pyrrolidinones: a new class of anticonvulsant agents. J Med Chem 39:1898-1906; De la Mora MP, Tapia R, 1973, Anticonvulsant effect of 5-ethyl,5-phenyl,2-pyrrolidinone and its possible relationship to γ-aminobutyric acid-dependent inhibitory mechanisms. Biochem Pharmacol 22:2635-2639).

Die Veröffentlichung Arzneimittelforschung 10, 1960, Seite 243-250 offenbart in Tabelle I Nr. XVII die Verbindung

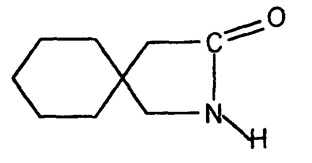

Diese Verbindung wird jedoch als nicht sonderlich wirksam angesehen, wie aus der Diskussion in der rechten Spalte auf Seite 249 dieser Veröffentlichung hervorgeht. Die Druckschrift enthält auch keinen Hinweis, diese Verbindung als Arzneimittel einzusetzen.

Es existiert zur Zeit kein zufriedenstellendes Arzneimittel, das den extrazellulären Glutamat-Spiegel wirksam senkt. Auch die bereits im Handel befindlichen Medikamente Riluzol und Lamotrigin sind als Hemmer der Glu-Freisetzung nur schwach wirksam und zeigen durch Metabolisierung oder ihren Wirkmechanismus Nebenwirkungen, die ihren therapeutischen Einsatz einschränken.

Es ist daher eine Aufgabe der Erfindung, neue Arzneistoffe bzw. Arzneimittel zur Verfügung zu stellen, die bei Erkrankungen wirksam sind, die auf einen erhöhten Glutamat-Spiegel zurückzuführen sind und die daher zur Prophylaxe und Behandlung von Erkrankungen des Zentralnervensystems wie Schlaganfall, Hypoglykämie, Hypoxie, Trauma und Epilepsie, Alzheimer'scher Erkrankung, Demenz bei AIDS, Amyotrophe Lateralsklerose, Morbus Parkinson und chronischer Alkoholismus eingesetzt werden können. Diese neuen Arzneimittel sollen die Nachteile der im Stand der Technik bekannten Arzneimittel nicht aufweisen.

Bevorzugt können die Arzneimittel die glutamaterge Transmission beeinflussen und den extrazellulären Glutamat-Spiegel verringern und/oder die Depolarisation und Übererregung postsynaptischer Zellen, beispielsweise durch präsynaptisch ausgeschüttetes Glutamat, verhindern.

Zur Lösung dieser Aufgabe wird erfindungsgemäß ein 2-Pyrrolidinon-Derivat der allgemeinen Formel 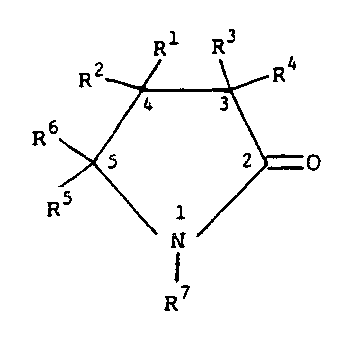 in der
R¹ und R² zusammen mit dem Kohlenstoffatom in 4-Stellung des Pyrrolidinonrings einen fünf- bis zehngliedrigen gesättigten oder ungesättigten Ring bilden, der neben Kohlenstoffatomen bis zu 2 Heteroatome ausgewählt aus Sauerstoff-, Schwefel- und Stickstoffatomen aufweisen kann und der unsubstituiert ist oder mit bis zu 3 Substituenten ausgewählt aus Hydroxylgruppen, Aminogruppen, C₁-C₄ Alkylresten, C₁-C₄ Alkoxyresten und C₁-C₄ Alkylaminoresten substituiert ist,
R³, R⁴, R⁵ und R⁶ jeweils unabhängig voneinander Wasserstoffatome, Halogenatome, Hydroxylgruppen, Aminogruppen, C₁-C₁₀ Alkylreste, C₁-C₁₀ Alkoxyreste, C₁-C₁₀ Alkylaminoreste oder C₆-C₁₀ Arylreste darstellen und
R⁷ ein Wasserstoffatom oder einen C₁-C₁₀ Alkylrest oder C₁-C₁₀ Acylrest darstellt,
pharmakologisch verträgliche Salze davon
zur Verwendung als therapeutischer Wirkstoff zur Verfügung gestellt.

Die Alkylreste ebenso wie die Alkylbestandteile der Alkoxyreste und Alkylaminoreste können geradkettig oder verzweigt sein.

Überraschend wurde gefunden, daß 2-Pyrrolidinon-Derivate, die in 4-Stellung einen Substituenten wie vorstehend definiert aufweisen, eine ausgezeichnete Wirkung bei der Verringerung des extrazellulären Glutamat-Spiegels aufweisen und daher zur Prophylaxe und Behandlung von Erkrankungen des Zentralnervensystems wie Schlaganfall, Hypoglykämie, Hypoxie, Trauma und Epilepsie, Alzheimer'scher Erkrankung, Demenz bei AIDS, Amyotrophe Lateralsklerose, Morbus Parkinson und chronischer Alkoholismus verwendet werden können. Die Verbindungen können ebenfalls bevorzugt die Depolarisation und Übererregung postsynaptischer Zellen, beispielsweise durch präsynaptisch ausgeschüttetes Glutamat, verhindern. Eine Substitution der erfindungsgemäßen 2-Pyrrolidinon-Derivate in Stellung 3 und 5 des Pyrrolidinonrings ist weitgehend unkritisch, soweit die Substitution in 4-Stellung des Pyrrolidinonrings gewährleistet ist. Bevorzugt sind 2-Pyrrolidinon-Derivate, die in 3- und 5-Stellung unsubstituiert sind oder ein oder zwei Alkylsubstituenten mit bis zu 10 Kohlenstoffatomen, bevorzugt bis zu 6 Kohlenstoffatomen und/oder ein oder zwei Arylsubstituenten mit 6 bis 10 Kohlenstoffatomen, bevorzugt Phenylgruppen, aufweisen.

Auch eine Substitution des Stickstoffatoms der 2-Pyrrolidinon-Derivate ist weitgehend unkritisch, bevorzugt ist jedoch das Stickstoffatom der erfindungsgemäßen 2-Pyrrolidinon-Derivate unsubstituiert oder mit einem C₁-C₆ Alkylrest oder einem C₁-C₆ Acylrest substituiert.

Wesentlich für die erfindungsgemäßen 2-Pyrrolidinon-Derivate ist die Substitution an 4-Stellung des Pyrrolidinonrings.

Wenn die Reste R¹ und R² wie in der vorstehend beschriebenen Ausführungsform der Erfindung verschieden sind, zeigen die beanspruchten 2-Pyrrolidinon-Derivate optische Isomerie. Erfindungsgemäß ist sowohl die reine R- als auch die reine S-Form der Pyrrolidinon-Derivate umfaßt, aber auch beliebige racemische Gemische aus der R- und S-Form.

Erfindungsgemäß besonders bevorzugt ist es, wenn die Reste R¹ und R² zusammen mit dem Kohlenstoffatom in 4-Stellung des Pyrrolidinonrings einen gesättigten oder ungesättigten fünfbis zehngliedrigen Ring bilden. Dieser Ring kann bis zu zwei Heteroatome ausgewählt aus Sauerstoff-, Schwefel- und Stickstoffatomen aufweisen und unsubstituiert sein oder mit bis zu drei Substituenten ausgewählt aus Hydroxylgruppen, Aminogruppen, C₁-C₄ Alkylresten, C₁-C₄ Alkoxyresten und C₁-C₄ Alkylaminoresten substituiert sein. Bevorzugt bilden die Reste R¹ und R² aber einen unsubstituierten Ring, der bevorzugt keine Heteroatome aufweist, bevorzugt gesättigt ist und besonders bevorzugt aus sechs Kohlenstoffatomen besteht, wobei das Kohlenstoffatom in 4-Stellung des 2-Pyrrolidinonrings eingeschlossen ist. Ein besonders bevorzugtes 2-Pyrrolidinon-Derivat der Erfindung ist das 8-Aza-spiro[5,4]decan-9-on (Gabapentin-Lactam).

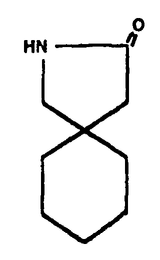

Sofern zumindest einer der Reste R³, R⁴, R⁵ und R⁶ von Wasserstoff verschieden ist, treten neben den optischen Isomeren (die erfindungsgemäß umfaßt sind) auch Strukturisomere auf. Sämtliche Strukturisomere sowie deren Gemische sind von den erfindungsgemäßen 2-Pyrrolidinon-Derivaten umfaßt.

Statt des 2-Pyrrolidinon-Derivats wie vorstehend definiert können auch pharmakologisch verträgliche Salze, insbesondere Säureadditionssalze, des 2-Pyrrolidinon-Derivats verwendet werden.

Die erfindungsgemäßen Verbindungen können auf an sich bekannte Art und Weise hergestellt werden. So ist das erfindungsgemäß besonders bevorzugte 8-Aza-spiro[5,4]decan-9-on bereits in der Literatur beschrieben (Kearney A.S., Mehta S.C., Radebaugh G.W., The effect of cyclodextrins on the rate of intramolecular lactamization of gabapentin in aqueous solution. International Journal of Pharmaceutics, 78 (1992), 25-34 sowie die bereits vorstehend diskutierte Literaturstelle Arzneimittelforschung 10, 1960, Seiten 243-250), eine Verwendung dieser Verbindung als therapeutischer Wirkstoff wird jedoch nicht vorgeschlagen. Das erfindungsgemäß bevorzugte 8-Aza-spiro[5,4]decan-9-on kann als Lactam der bekannten Verbindung Gabapentin angesehen werden und z.B. durch Bestrahlung einer phosphatgepufferten wäßrigen Gabapentinlösung mit ultraviolettem Licht hergestellt werden. Durch Lactamisierung entsprechend substituierter Gabapentinderivate können substituierte Derivate des 8-Azaspiro[5,4]decan-9-ons hergestellt werden. Bevorzugt sind solche Derivate, die einen C₁-C₄ Alkylrest, ein Halogen, eine Hydroxylgruppe oder eine Aminogruppe, bevorzugt einen C₁-C₄ Alkylrest oder ein Halogenatom, aufweisen.

Die Verbindung kann auch nach folgendem Schema hergestellt werden.

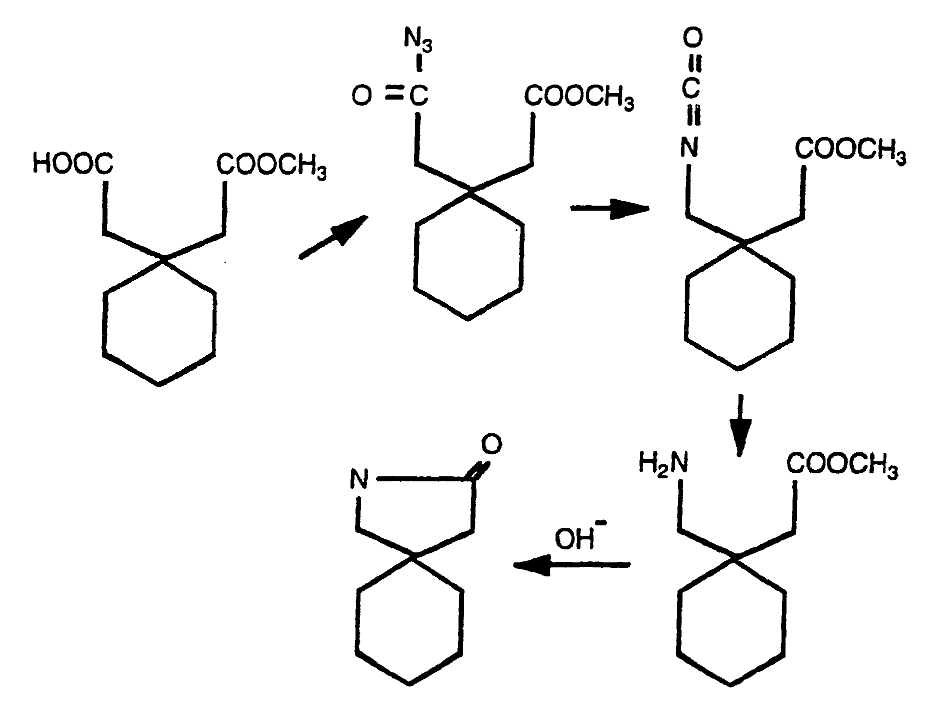

1,1-Cyclohexandiessigsäuremonomethylester wird über das entsprechende Säurechlorid zu dem Azid umgesetzt. Das Azid wird nach dem Curtius-Verfahren zum Isocyanat abgebaut. Anschließend wird das Isocyanat zu 1-Methylamino-1-essigsäurecyclohexanmethylester hydrolisiert. Erhitzen dieser Substanz in alkalischem Methanol unter Rückfluß über drei Tage ergibt 8-Aza-spiro[5,4]decan-9-on oder Gabapentin-Lactam.

Eine allgemeine Synthesevorschrift für die erfindungsgemäßen 4-substituierten 2-Pyrrolidinone geht in Anlehnung an die von Andruszkiewicz und Silverman veröffentlichte Synthese von 3-substituierten GABA-Derivaten (R. Andruszkiewicz and R.B. Silverman, Synthesis 953-955 (1989)) von entsprechend substituierten α,β-ungesättigten Carbonsäureestern (1) aus, die u. a. durch eine Reformatzky-Reaktion zugänglich sind. Nach Umsetzung mit Nitromethan erhält man in einer Michael-Addition eine Nitroverbindung (2), die nach Reduktion mit elementarem Wasserstoff in die entsprechende Aminoverbindung (3) übergeht. Nach Esterspaltung und der Aktivierung der Carboxylatfunktion durch eine gut austretende Gruppe (z. B. Überführen in das Carbonsäurehalogenid) erhält man unter Zyklisierung das entsprechende 4-substituierte 2-Pyrrolidinon.

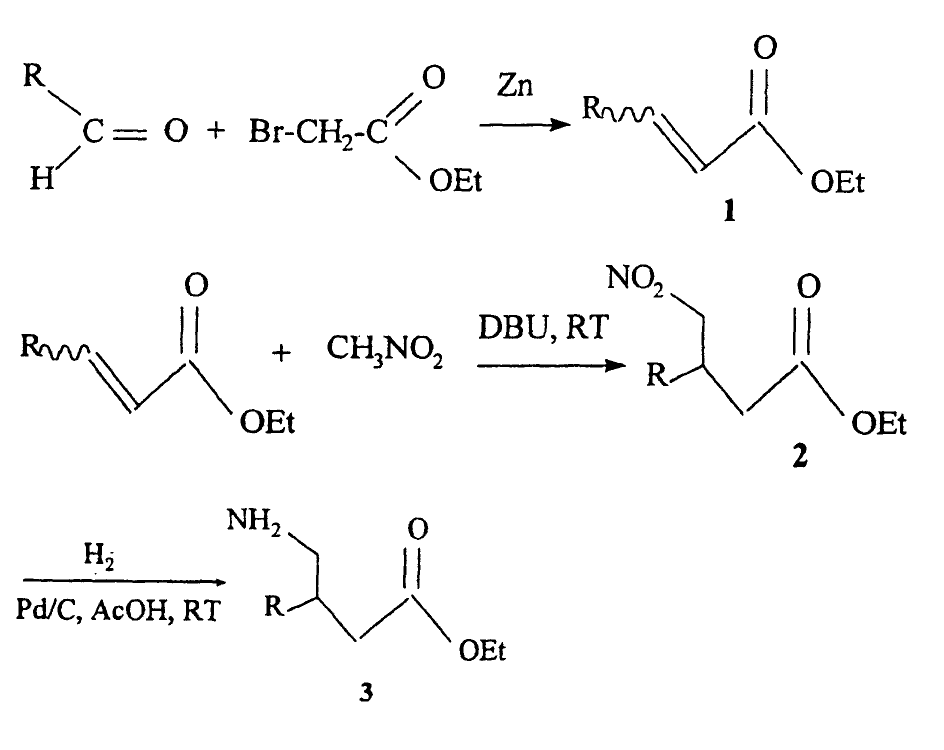

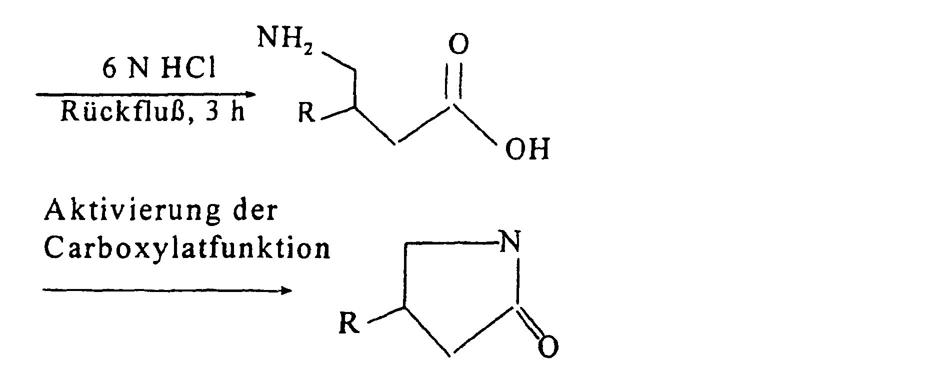

Die erfindungsgemäßen Zusammensetzungen können auf an sich bekannte Art und Weise zu Arzneimitteln für Säuger, bevorzugt Menschen, formuliert werden. In den Arzneimitteln liegen die erfindungsgemäßen Zusammensetzungen im Gemisch mit einem pharmazeutischen organischen oder anorganischen Träger, der für enterale oder parenterale Verabreichungen geeignet ist, vor. Die orale Verabreichung der erfindungsgemäßen Zusammensetzungen über Tabletten, Kapseln, Pulver oder in flüssiger Form, wie als Suspensionen, in Lösung, als Emulsion oder als Sirup ist besonders bevorzugt.

Bei der Formulierung als Tabletten werden übliche Arzneimittelträger wie Natriumcitrat, Lactose, microkristalline Cellulose und Stärke, Schmiermittel wie wasserfreie Kieselsäure, hydriertes Castoröl, Magnesiumstearat, Natriumlaurylsulfat und Talk, sowie Bindemittel wie Stärkepaste, Glucose, Lactose, Gummi-Arabicum, Mannit, Magnesiumtrisilicat und Talk verwendet. Wenn die erfindungsgemäßen Zusammensetzungen über Flüssigkeiten verabreicht werden sollen, können übliche flüssige Träger verwendet werden.

Möglich ist ebenfalls eine Formulierung für Injektionen und Infusionen oder als Suppositorien, wie es auf dem Fachgebiet bekannt und in einschlägigen Standardwerken beschrieben ist.

Die erfindungsgemäßen Zusammensetzungen können ebenfalls auf an sich bekannte Art und Weise als Depotformulierungen oder zu Arzneimitteln mit verzögerter oder hinhaltender Freisetzung formuliert werden.

Die Dosisform der erfindungsgemäßen Zusammensetzungen hängt von der speziellen Zusammensetzung und weiteren Faktoren ab und kann von einem Fachmann aufgrund des Zustands des zu behandelnden Patienten, der Schwere und Art der zu behandelnden Krankheit, möglicher Nebenwirkungen der Verbindungen, usw., bestimmt werden.

Die Beispiele erläutern die Erfindung.

### Beispiel 1

Eine Lösung aus Gabapentin (100µM) in physiologischem Puffer (Zusammensetzung nachstehend) mit einem pH-Wert von 7,4 wurde hergestellt und mit ultraviolettem Licht (260 und 330 Nanometer) zwei Stunden bei 37°C bestrahlt. Wie in Kearney A.S. et al., International Journal of Pharmaceutics, 78 (1992), 25-34 beschrieben, entsteht hierbei langsam das Lactam 8-Aza-spiro[5,4]decan-9-on. Aufgrund der langsamen Reaktionsgeschwindigkeit wird gemäß Kearney et al. (1992) eine Ausbeute von etwa 1% angenommen, was einer 1µM Lösung entspricht.

Die Wirksamkeit dieser Lösung zur Verringerung des extrazellulären Glutamat-Spiegels wurde anhand von Superfusionsexperimenten an Hirnschnitten der Ratte gezeigt. Hierzu wurden 350 µm dicke Schnitte aus dem Striatum der Ratte verwendet. Je zwei Schnitte werden in eine Superfusionskammer mit extrem kleinem Totvolumen, etwa 100 µl, eingesetzt. Anschließend werden sie bei 37°C mit physiologischem Puffer (Konzentrationen in mM: NaCl 121, KCl 1,8, CaCl₂ 1,3, MgSO₄ 1,2, NaHCO₃ 25, KH₂PO₄ 1,2, Glucose 11, pH 7,4, 95% O₂/5% CO₂gesättigt) umspült. Nach einer Vorperfusion, die vor allem dazu dient, Gewebepartikel und Aminosäuren, die durch Verletzung des Gewebes freigesetzt wurden, zu entfernen, werden Superfusat-Fraktionen mit einer Flußrate von 400 bis 800 µl pro fünf Minuten gesammelt. Sechs der 12 zur Verfügung stehenden Kammern werden ab Beginn der Vorperfusion fortwährend mit der etwa 1µM Lösung von 8-Aza-spiro[5,4]decan-9-on durchspült, die restlichen Kammern, die mit Puffer durchspült werden, dienen als Kontrolle. Die erhaltenen Superfusate werden filtriert (0,45 µM Porengröße) und die Aminosäurekonzentrationen über HPLC bestimmt. Einzelheiten des Verfahrens sind beispielsweise in R. Knörle et al., Neuroscience Letters 221 (1997), 169-172 beschrieben.

Die Ergebnisse zeigen eine deutliche Reduktion der extrazellulären Glutamatkonzentration durch das 8-Azaspiro[5,4]decan-9-on. Bei den Proben, die die erfindungsgemäße Verbindung enthalten, wurde 0,12 µM Glutamat, CI₉₅ = [0,10, 0,13] gefunden, während in den Kontrollversuchen 0,24 µM Glutamat, CI₉₅ = [0,19, 0,29] gefunden wurde.

### Beispiel 2

Die neuroprotektive Wirkung der erfindungsgemäßen Verbindungen wurde *in vivo* nachgewiesen auf Grundlage der Veröffentlichung in Invest Ophthalmol Vis Sci 39: 1063-1066, 1998. Folgende Ergebnisse wurden gefunden:

10 Ratten wurden zu Beginn einer einseitigen einstündigen intraokularen Druckerhöhung mit 0,9% NaCl i.p. behandelt. Nach 6 Stunden wurde die Behandlung erneut mit 0,9% NaCl i.p. durchgeführt. Nach 14 Tagen wurden die Ratten getötet, und die retinalen Ganglienzellen wurden untersucht. Im Vergleich zu dem jeweiligen Kontrollauge hatten nur 17,4% (±4%) der retinalen Ganglienzellen überlebt. Die Ratten verhielten sich in den 14 Tagen nach dem Versuch völlig unauffällig, kein Tier starb.

In der Versuchsgruppe aus ebenfalls 10 Ratten wurde zu Beginn einer einseitigen einstündigen intraokularen Druckerhöhung 50 mg/kg 8-Aza-spiro[5,4]decan-9-on i.p. verabreicht. Die Verbindung wurde 6 Stunden nach der intraokularen Druckerhöhung erneut in einer Dosis von 50 mg/kg verabreicht. Nach 14 Tagen wurden die Ratten getötet, und die retinalen Ganglienzellen wurden untersucht. Es zeigte sich, daß im Vergleich zum jeweiligen Kontrollauge 35% (±7%) der retinalen Ganglienzellen überlebten. Die Ratten verhielten sich in den 14 Tagen nach dem Versuch völlig unauffällig, kein Tier starb.

Dieser Versuch zeigt deutlich die Steigerung der überlebenden Retinaganglienzellen nach druckbedingter retinaler Ischämie von 17,4% (±4%) auf 35,0% (±7%). Die Neurodegeneration in diesem Glaucom-Modell beruht auf einer NMDArezeptorvermittelten Glu-Toxizität.

### Beispiel 3

In einem weiteren *in vitro* Versuch konnte gezeigt werden, daß der antischämiche Wirkmechanismus der erfindungsgemäßen Verbindungen auf einer Reduktion des ausgeschütteten Glu beruht.

350 µm dicke Hippocampusschnitte der Ratte wurden bei 37°C 60 Minuten in 3 ml Medium inkubiert. Das Medium enthielt zur Markierung des endogenen Gln-Pools 2 µM [³H]-Gln. Anschließend synthetisieren die Schnitte Glu. Die Schnitte wurden gewaschen und dann mit einem Puffer 50 Minuten einer Superfusion ausgesetzt. Im 5 Minuten Abstand wurden insgesamt 15 Superfusionsproben durch Anion-Austausch-Chromatographie [³H]-Glu extrahiert und mit Flüssigscintillationszählung gemessen. Aus dem [³H]-Glu, das aus den Superfusionsproben und aus den Gewebeschnitten nach der Superfusion extrahiert worden war, wurden die fraktionellen Geschwindigkeiten der [³H]-Glu-Freisetzung berechnet.

Gabapentin-Lactam wurde zunächst in einem Modell überprüft, bei dem die Freisetzung durch elektrisches Stimulieren hervorgerufen wurde. Die Freisetzung spricht in diesem Modell sowohl auf Tetrodotoxin als auch auf extrazelluläre Ca⁺⁺-Ionen an. Die Schnitte wurden nach der Inkubation mit tritiertem Gln einer Superfusion ausgesetzt und zweimal elektrisch stimuliert, wobei 540 Pulse von 4 ms, 6 Hz und 60 mA eingesetzt wurden. Die Superfusion wurde bei Raumtemperatur, nicht bei 37°C, durchgeführt. Ferner war 100 µM des Glu-Aufnahmeblockers PDC vorhanden, damit die erneute Aufnahme von freigesetztem Glu reduziert wird. TTX oder Gabapentin-Lactam wurde von 15 Minuten vor der zweiten Stimulation an zugesetzt. Es wurde gefunden, daß die elektrisch hervorgerufene Freisetzung von Glu teilweise durch TTX inhibiert wird, nicht jedoch durch Gabapentin-Lactam. Die quasi-physiologische Freisetzung von Glu wird daher durch Gabapentin-Lactam nicht beeinflußt.

Das Modell des endogen gebildeten [³H]-Glu wurde ebenfalls zur Erzeugung von ischämischen Bedingungen *in vitro* verwendet. Zur "quasi-ischämisch" Stellung eines Schnitts wurde während der Superfusion Sauerstoff und Glucose durch Stickstoff und Saccharose ersetzt. Saccharose wurde anstelle von 11 mM Glucose verwendet, um die Osmolarität der Superfusionsflüssigkeit zu erhalten. Es ist bekannt, daß Saccharose *in vitro* nicht in Glucose und Fructose gespalten werden kann. Anders als bei den Versuchen, bei denen die [³H]-Glu-Freisetzung elektrisch hervorgerufen wurde, und um eine Ischämie so gut wie möglich zu simulieren, wurde die [³H]-Glu-Freisetzung während der Inkubation und Superfusion bei 37°C durchgeführt. Es wurde festgestellt, daß die übliche hohe Konzentration des Glu-Aufnahme-Inhibitors PDC, nämlich 100 µM, die Antwort auf die Ischämie merklich verringerte. Daher wurden alle Versuche in Gegenwart von nur 3 µM PDC durchgeführt, was den Effekt der Ischämie deutlich erhöhte.

Es wurde gefunden, daß in diesem *in vitro* Ischämie-Modell Gabapentin-Lactam den neurotoxischen [³H]-Glu-Ausstoß signifikant verringert.

Die Verbindungen verhindern damit *in vitro* den starken Anstieg von extrazellulärem Tritium-Glu in (mit Tritium-Glutamin vorbeladenen) Hippocampusschnitten der Ratte nach Ersatz von O₂ durch N₂ und von Glucose durch Succhrose eindeutig und zwar um circa 50%. Die quasi-physiologische aktionspotentialvermittelte Glu-Freisetzung wurde durch Gabapentinlactam jedoch nicht beeinflußt.

### Vergleichsbeispiel 1

Beispiel 1 wurde wiederholt mit der Abwandlung, daß die Gabapentin-haltige Lösung nicht mit ultraviolettem Licht bestrahlt wurde, sondern unmittelbar in dem Versuch verwendet wurde. Die Vergleichslösung enthält also ausschließlich Gabapentin, jedoch kein 8-Aza-spiro[5,4]decan-9-on. Bei den entsprechenden Versuchen konnte kein Unterschied zwischen den Proben, die mit der Gabapentinlösung behandelt wurden und den Vergleichsproben gefunden werden.

### Vergleichsbeispiel 2

Beispiel 2 vorstehend wurde wiederholt, jedoch wurde statt 8-Aza-spiro[5,4]decan-9-on die strukturell ähnliche Verbindung Gabapentin eingesetzt. Es zeigte sich, daß Gabapentin keinerlei Schutzwirkung gegen Neurodegeneration in diesem Glaucom-Modell zeigte.

## Patentansprüche

1. 2-Pyrrolidinon-Derivat der allgemeinen Formel 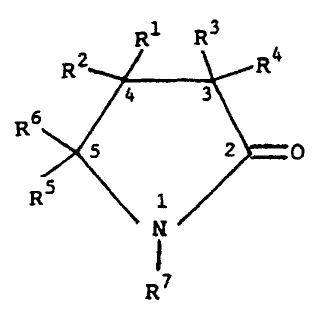 in der
R¹ und R² zusammen mit dem Kohlenstoffatom in 4-Stellung des Pyrrolidinonrings einen fünf- bis zehngliedrigen gesättigten oder ungesättigten Ring bilden, der neben Kohlenstoffatomen bis zu 2 Heteroatome ausgewählt aus Sauerstoff-, Schwefel- und Stickstoffatomen aufweisen kann und der unsubstituiert ist oder mit bis zu 3 Substituenten ausgewählt aus Hydroxylgruppen, Aminogruppen, C₁-C₄ Alkylresten, C₁-C₄ Alkoxyresten und C₁-C₄ Alkylaminoresten substituiert ist,
R³, R⁴, R⁵ und R⁶ jeweils unabhängig voneinander Wasserstoffatome, Halogenatome, Hydroxylgruppen, Aminogruppen, C₁-C₁₀ Alkylreste, C₁-C₁₀ Alkoxyreste, C₁-C₁₀ Alkylaminoreste oder C₆-C₁₀ Arylreste darstellen und
R⁷ ein Wasserstoffatom oder einen C₁-C₁₀ Alkylrest oder C₁-C₁₀ Acylrest darstellt,
oder eines pharmakologisch verträglichen Salzes davon
zur Verwendung als therapeutischer Wirkstoff.

2. 2-Pyrrolidinon-Derivat nach Anspruch 1, wobei die Reste R³, R⁴, R⁵ und R⁶ Wasserstoffatome darstellen.

3. 2-Pyrrolidinon-Derivat nach Anspruch 1 oder 2, wobei die Reste R¹ und R² zusammen mit dem Kohlenstoffatom in 4-Stellung des Pyrrolidinonrings einen sechsgliedrigen gesättigten Kohlenwasserstoffring bilden.

4. 2-Pyrrolidinon-Derivat nach Anspruch 3, nämlich 8-Aza-spiro[5,4]decan-9-on.

5. Verwendung eines 2-Pyrrolidinon-Derivats der allgemeinen Formel 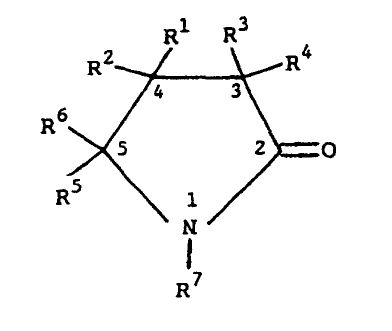 in der
R¹ und R² zusammen mit dem Kohlenstoffatom in 4-Stellung des Pyrrolidinonrings einen fünf- bis zehngliedrigen gesättigten oder ungesättigten Ring bilden, der neben Kohlenstoffatomen bis zu 2 Heteroatome ausgewählt aus Sauerstoff-, Schwefel- und Stickstoffatomen aufweisen kann und der unsubstituiert ist oder mit bis zu 3 Substituenten ausgewählt aus Hydroxylgruppen, Aminogruppen, C₁-C₄ Alkylresten, C₁-C₄ Alkoxyresten und C₁-C₄ Alkylaminoresten substituiert ist,
R³, R⁴, R⁵ und R⁶ jeweils unabhängig voneinander Wasserstoffatome, Halogenatome, Hydroxylgruppen, Aminogruppen, C₁-C₁₀ Alkylreste, C₁-C₁₀ Alkoxyreste, C₁-C₁₀ Alkylaminoreste oder C₈-C₁₀ Arylreste darstellen und
R⁷ ein Wasserstoffatom oder einen C₁-C₁₀ Alkylrest oder C₁-C₁₀ Acylrest darstellt oder
eines pharmakologisch verträglichen Salzes davon,
zur Herstellung eines Arzneimittels zur Behandlung und/oder Vorbeugung von Erkrankungen, die auf einem erhöhten extrazellulären Glutamat-Spiegel beruhen.

6. Verwendung nach Anspruch 5, wobei die Reste R³, R⁴, R⁵ und R⁶ Wasserstoffatome darstellen.

7. Verwendung nach Anspruch 5 oder 6, wobei die Reste R¹ und R² zusammen mit dem Kohlenstoffatom in 4-Stellung des Pyrrolidinonrings einen sechsgliedrigen gesättigten Kohlenwasserstoffring bilden, der unsubstituiert ist oder einen Substituenten, ausgewählt aus einer Hydroxylgruppe, einer Aminogruppe, einem Halogenatom oder einem C₁-C₄ Alkylrest, aufweist.

8. Verwendung nach Anspruch 7, bei der das 2-Pyrrolidinon-Derivat 8-Azaspiro[5,4]decan-9-on ist.

9. Verwendung eines 2-Pyrrolidinon-Derivats wie in einem der Ansprüche 5 bis 8 definiert, wobei das Arzneimittel zur Vorbeugung vor und zur Behandlung von Schlaganfall dient.

## Claims

1. A 2-pyrrolidinone derivative of the general formula 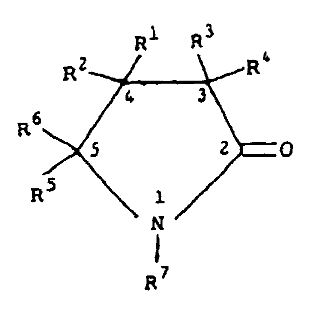 in which
R¹ and R² together with the carbon atom in position 4 of the pyrrolidinone ring form a five- to ten-membered saturated or unsaturated ring which, besides carbon atoms, may have up to 2 heteroatoms selected from oxygen, sulfur and nitrogen atoms, and which is unsubstituted or is substituted by up to 3 substituents selected from hydroxyl groups, amino groups, C₁-C₄ alkyl radicals, C₁-C₄ alkoxy radicals and C₁-C₄ alkylamino radicals,
R³, R⁴, R⁵ and R⁶ are, each independently of one another, hydrogen atoms, halogen atoms, hydroxyl groups, amino groups, C₁-C₁₀ alkyl radicals, C₁-C₁₀ alkoxy radicals, C₁-C₁₀ alkylamino radicals or C₆-C₁₀ aryl radicals, and
R⁷ is a hydrogen atom or a C₁-C₁₀ alkyl radical or C₁-C₁₀ acyl radical,
or of a pharmacologically acceptable salt thereof
for use as therapeutic active ingredient.

2. A 2-pyrrolidinone derivative as claimed in claim 1, where the radicals R³, R⁴, R⁵ and R⁶ are hydrogen atoms.

3. A 2-pyrrolidinone derivative as claimed in claim 1 or 2, where the radicals R¹ and R² form, together with the carbon atom in position 4 of the pyrrolidinone ring, a six-membered saturated hydrocarbon ring.

4. A 2-pyrrolidinone derivative as claimed in claim 3, namely 8-azaspiro[5.4]decan-9-one.

5. The use of a 2-pyrrolidinone derivative of the general formula 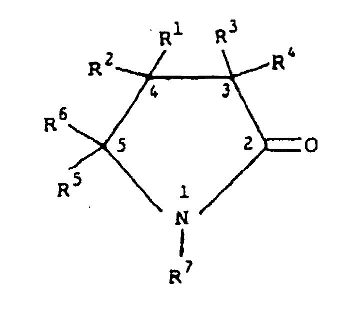 in which
R¹ and R² together with the carbon atom in position 4 of the pyrrolidinone ring form a five- to ten-membered saturated or unsaturated ring which, besides carbon atoms, may have up to 2 heteroatoms selected from oxygen, sulfur and nitrogen atoms, and which is unsubstituted or is substituted by up to 3 substituents selected from hydroxyl groups, amino groups, C₁-C₄ alkyl radicals, C₁-C₄ alkoxy radicals and C₁-C₄ alkylamino radicals,
R³, R⁴, R⁵ and R⁶ are, each independently of one another, hydrogen atoms, halogen atoms, hydroxyl groups, amino groups, C₁-C₁₀ alkyl radicals, C₁-C₁₀ alkoxy radicals, C₁-C₁₀ alkylamino radicals or C₆-C₁₀ aryl radicals, and
R⁷ is a hydrogen atom or a C₁-C₁₀ alkyl radical or C₁-C₁₀ acyl radical, or
of a pharmacologically acceptable salt thereof
for the production of a pharmaceutical composition for the treatment and/or prevention of disorders based on an elevated extracellular glutamate level.

6. The use as claimed in claim 5, where the radicals R³, R⁴, R⁵ and R⁶ are hydrogen atoms.

7. The use as claimed in claim 5 or 6, where the radicals R¹ and R² form, together with the carbon atom in position 4 of the pyrrolidinone ring, a six-membered saturated hydrocarbon ring which is unsubstituted or has a substituent selected from a hydroxyl group, an amino group, a halogen atom or a C₁-C₄ alkyl radical.

8. The use as claimed in claim 7, wherein the 2-pyrrolidinone derivative is 8-azaspiro[5,4]decan-9-one.

9. The use of a 2-pyrrolidinone derivative as defined in any of claims 5 to 8, where the pharmaceutical composition is used for the prevention and for the treatment of stroke.

## Revendications

1. Dérivé de 2-pyrrolidinone de formule générale 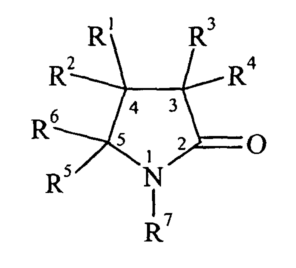 dans laquelle
R¹ et R², avec l'atome de carbone en position 4 du noyau pyrrolidinone, forment un noyau saturé ou insaturé à cinq à dix chaînons, qui outre des atomes de carbone peut comporter jusqu'à 2 hétéroatomes choisis parmi les atomes d'oxygène, de soufre et d'azote, et qui est non substitué, ou est substitué par jusqu'à 3 substituants choisis parmi les groupes hydroxyle, les groupes amino, les radicaux alkyle en C₁-C₄, les radicaux alcoxy en C₁-C₄ et les radicaux alkylamino en C₁-C₄,
chacun des radicaux R³, R⁴, R⁵ et R⁶ est, indépendamment des autres, un atome d'hydrogène, un atome d'halogène, un groupe hydroxyle, un groupe amino, un radical alkyle en C₁-C₁₀, un radical alcoxy en C₁-C₁₀, un radical alkylamino en C₁-C₁₀ ou un radical aryle en C₆-C₁₀, et
R⁷ est un atome d'hydrogène ou un radical alkyle en C₁-C₁₀, ou un radical acyle en C₁-C₁₀,
ou l'un de ses sels acceptables d'un point de vue pharmacologique,
pour utilisation en tant que principe actif thérapeutique.

2. Dérivé de 2-pyrrolidinone selon la revendication 1, dans lequel les radicaux R³, R⁴, R⁵ et R⁶ représentent des atomes d'hydrogène.

3. Dérivé de 2-pyrrolidinone selon la revendication 1 ou 2, dans lequel les radicaux R¹ et R², avec l'atome de carbone en position 4 du noyau pyrrolidinone, forment un noyau hydrocarboné saturé à six chaînons.

4. Dérivé de 2-pyrrolidinone selon la revendication 3, à savoir la 8-aza-spiro[5,4]décane-9-one.

5. Utilisation d'un dérivé de 2-pyrrolidinone de formule générale 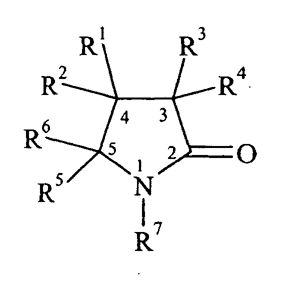 dans laquelle
R¹ et R², avec l'atome de carbone en position 4 du noyau pyrrolidinone, forment un noyau saturé ou insaturé à cinq à dix chaînons, qui outre des atomes de carbone peut comporter jusqu'à 2 hétéroatomes choisis parmi les atomes d'oxygène, de soufre et d'azote, et qui est non substitué, ou est substitué par jusqu'à 3 substituants choisis parmi les groupes hydroxyle, les groupes amino, les radicaux alkyle en C₁-C₄, les radicaux alcoxy en C₁-C₄ et les radicaux alkylamino en C₁-C₄,
chacun des radicaux R³, R⁴, R⁵ et R⁶ est, indépendamment des autres, un atome d'hydrogène, un atome d'halogène, un groupe hydroxyle, un groupe amino, un radical alkyle en C₁-C₁₀, un radical alcoxy en C₁-C₁₀, un radical alkylamino en C₁-C₁₀ ou un radical aryle en C₆-C₁₀, et
R⁷ est un atome d'hydrogène ou un radical alkyle en C₁-C₁₀, ou un radical acyle en C₁-C₁₀, ou d'un sel acceptable d'un point de vue pharmaceutique de ce dérivé,
pour préparer un médicament destiné au traitement et/ou à la prévention de maladies qui se fondent sur une augmentation du taux extracellulaire du glutamate.

6. Utilisation selon la revendication 3, pour laquelle les radicaux R³, R⁴, R⁵ et R⁶ sont des atomes d'hydrogène.

7. Utilisation selon la revendication 5 ou 6, pour laquelle les radicaux R¹ et R², avec l'atome de carbone en position 4 du noyau pyrrolidinone, forment un noyau hydrocarboné saturé à six chaînons, qui est non substitué ou comporte un substituant choisi parmi un groupe hydroxyle, un groupe amino, un atome d'halogène ou un radical alkyle en C₁-C₄.

8. Utilisation selon la revendication 7, pour laquelle le dérivé de 2-pyrrolidinone est la 8-aza-spiro[5,4]décane-9-one.

9. Utilisation d'un dérivé de 2-pyrrolidinone selon l'une des revendications 5 à 8, le médicament servant à la prévention et au traitement de l'accident vasculaire cérébral.
